# EUROPEAN PATENT APPLICATION

(11) **EP 4 039 828 A1**
(43) Date of publication of application: **10.08.2022**
(21) Application number: 20871546.6
(22) Date of filing: 29.09.2020
(51) Int. Cl.: C12Q 1/6883, G01N 33/68

(54) **BIOMARKER COMPOSITION FOR PREDICTING THERAPEUTIC EFFECT OF MESENCHYMAL STEM CELLS ON SYSTEMIC LUPUS ERYTHEMATOSUS**

(30) Priority: 02.10.2019 KR 20190122191
(71) Applicant: Corestem Co., Ltd., Seongnam-si, Gyeonggi-do 13486 (KR); Chungbuk National University Industry-Academic Cooperation Foundation, Cheongju-si, Chungcheongbuk-do 28644 (KR)
(72) Inventor: KIM, Kyung Suk, Seoul 02838 (KR); HAN, Sang Bae, Cheongju-si, Chungcheongbuk-do 28692 (KR); LEE, Tae Yong, Yongin-si, Gyeonggi-do 17128 (KR); KIM, Hyung Sook, Cheongju-si, Chungcheongbuk-do 28109 (KR)
(74) Representative: HGF
(86) International application number: PCT/KR2020/013352
(87) International publication number: WO 2021/066526

(57) **Abstract**

The present invention relates to a biomarker composition for predicting the therapeutic effect of mesenchymal stem cells on systemic lupus erythematosus.

## Description

### Technical Field

This application claims priority benefit to Korean Patent Application No. 10-2019-0122191 filed on October 2, 2019, which is incorporated herein by reference in its entirety.

The present invention relates to a biomarker composition for predicting the therapeutic effect of mesenchymal stem cells on systemic lupus erythematosus.

### Background Art

Systemic lupus erythematosus (SLE), also called lupus, is a prototypical systemic autoimmune disease. Systemic lupus erythematosus is an autoimmune disease characterized by the presence of a series of antibodies, including antibodies to DNA, antibodies to nuclear antigens, and autoantibodies to ribonucleoproteins. Systemic lupus erythematosus includes consistent symptoms and signs, musculoskeletal, skin, kidney, gastrointestinal, lung, cardiac, reticuloendothelial, hematological and neuropsychiatric manifestations. Skin manifestations are the most common in systemic lupus erythematosus. The progression of systemic lupus erythematosus is associated with general clinical symptoms and loss for tissues and organs caused by precipitation of immune complexes. Similar to other autoimmune symptoms, the etiology of systemic lupus erythematosus is multifactorial, involving genetic, environmental, hormonal and immunological factors.

Lupus nephritis is a glomerulonephritis associated with systemic lupus erythematosus, and the deposition of immune complexes including anti-dsDNA and complement plays an important pathophysiological role. The amount of proteinuria reflects the size of the peripheral glomerular capillary ring and tends to increase with intervascular proliferation and membraneous nephropathy. Proliferative glomerulonephritis (Classes 2 and 3) is the most severe form of lupus nephritis.

Mesenchymal stem cells are highly proliferative adherent cells having multipotency capable of differentiating into bone, cartilage, fat and the like, and are known to have antiinflammatory and immunomodulatory abilities. Mesenchymal stem cells exhibit immunosuppressive effects such as inhibition of proliferation and differentiation of T cells and B cells, and inhibition of functions of immune cells such as dendritic cells, natural killer (NK) cells and macrophages. Recently, a study of increasing the engraftment rate of hematopoietic stem cells by transplanting mesenchymal stem cells together with hematopoietic stem cells has been reported. It has been reported that mesenchymal stem cells reduce inflammation and inhibit autoimmune hyperreactivity in diseases such as graft versus host disease (GVHD), collagen-induced arthritis (CIA), experimental autoimmune encephalomyelitis (EAE), sepsis, acute pancreatitis (AP), colitis, multiple sclerosis (MS) and rheumatoid arthritis. It has been disclosed that mesenchymal stem cells also reduce inflammation and have therapeutic effects in systemic lupus erythematosus (SLE).

Previously, in order to confirm the therapeutic effect of mesenchymal stem cells in systemic lupus erythematosus disease animal models, survival rate, anti-dsDNA Ab in blood, total IgG in blood, and proteinuria were measured. However, the existing biomarkers for predicting the therapeutic effect have limitations in that the measurement thereof is difficult, it can be measured after the disease has progressed considerably, and the accuracy is low. Therefore, there is a need for research on new biomarkers.

### Detailed Description of Invention

### Technical Problem

The present inventors measured the therapeutic effect of mesenchymal stem cells in a systemic lupus erythematosus disease animal model using the existing biomarkers (survival rate, anti-dsDNA Ab in blood, total IgG in blood, and proteinuria), and confirmed that new biomarkers (chemokine, cytokine, and CHI3L1) may be also measured. Based on the above, the present inventors completed the present invention.

More specifically, it was confirmed that when systemic lupus erythematosus progressed in MRL/lpr mice, anti-dsDNAAb in blood, total IgG in blood, and proteinuria levels were increased, and ultimately the mice died. It was confirmed that when mesenchymal stem cells were administered to these mice, anti-dsDNA Ab in blood, total IgG in blood, and proteinuria levels were reduced. Therefore, it was confirmed that these existing biomarkers might confirm the therapeutic effect of mesenchymal stem cells on systemic lupus erythematosus.

Furthermore, the present inventors confirmed that when systemic lupus erythematosus progressed in MRL/lpr mice, the expression levels of various chemokines, cytokines, and CHI3L1 were changed (increased or reduced) and were regulated by the administration of mesenchymal stem cells. Typically, it was confirmed that mesenchymal stem cells reduced the expression of CCL2, CCL3, CCL4, CCL5, CCL7, CCL11, CCL17, CCL19, CXCL9, CXCL10, and CXCL11 in the kidney, increased the expression level of CCL8, reduced the expression of IFNs, IL-1β, IL-6, TNF-α, IL-2, IL-4, IL-10, IL-17, and TGF-α, and reduced the expression of CHI3L1.

Through this, it was confirmed that in order to predict the therapeutic effect of mesenchymal stem cells on systemic lupus erythematosus, CCL2, CCL5, CCL8, IL-1β, and CHI3L1 may be utilized along with the existing biomarkers such as anti-dsDNA Ab in blood, total IgG in blood, and proteinuria levels.

### Solution to Problem

The present invention may provide a biomarker composition for predicting the therapeutic effect of mesenchymal stem cells on systemic lupus erythematosus, comprising an agent for measuring an expression level of the mRNA or protein of CCL2, CCL3, CCL4, CCL5, CCL8, CCL7, CCL11, CCL17, CCL19, CXCL9, CXCL10, CXCL11, IFNs, IL-1β, IL-6, TNF-α, IL-2, IL-4, IL-10, IL-17, TGF-α or CHI3L1.

The agent for measuring the mRNA expression level may be a primer pair, a probe or an antisense nucleotide that specifically binds to the mRNA.

The agent for measuring the protein expression level may be an antibody that specifically binds to the protein or a fragment of the protein.

The present invention may also provide a kit for predicting the therapeutic effect of mesenchymal stem cells on systemic lupus erythematosus, comprising the composition described above.

The kit may be a RT-PCR (reverse transcription polymerase chain reaction) kit, a DNA chip kit, an ELISA (enzyme-linked immunosorbent assay) kit, a protein chip kit, a rapid kit or an MRM (multiple reaction monitoring) kit.

The present invention may also provide a method of providing information for predicting the therapeutic effect of mesenchymal stem cells on systemic lupus erythematosus, comprising: (a) measuring an expression level of the mRNA or protein of CCL2, CCL3, CCL4, CCL5, CCL8, CCL7, CCL11, CCL17, CCL19, CXCL9, CXCL10, CXCL11, IFNs, IL-1β, IL-6, TNF-α, IL-2, IL-4, IL-10, IL-17, TGF-α or CHI3L1 from a sample; and (b) comparing the measured expression level to the expression level of a control group.

The sample may be obtained from a patient with systemic lupus erythematosus who has been treated with mesenchymal stem cells.

The measurement of the mRNA expression level may use reverse transcription polymerase chain reaction (RT-PCR), competitive reverse transcription polymerase chain reaction (competitive RT-PCR), real time reverse transcription polymerase chain reaction (real time quantitative RT-PCR), quantitative polymerase chain reaction (quantitative RT-PCR), RNase protection method, Northern blotting or DNA chip technology.

The measurement of the protein expression level may use Western blotting, immunohistochemical staining, immunoprecipitation assay, complement fixation assay or immunofluorescence.

The method may determine that the mesenchymal stem cells have the therapeutic effect or the excellent therapeutic effect when the gene expression level of CCL2, CCL3, CCL4, CCL5, CCL7, CCL11, CCL17, CCL19, CXCL9, CXCL10, CXCL11, IFNs, IL-1β, IL-6, TNF-α, IL-2, IL-4, IL-10, IL-17, TGF-α or CHI3L1 is measured to be low compared to that of the control sample.

The method may determine that the mesenchymal stem cells have the therapeutic effect or the excellent therapeutic effect when the gene expression level of CCL8 is measured to be high compared to that of the control sample.

### Effects of Invention

According to the present invention, CCL2, CCL3, CCL4, CCL5, CCL8, CCL7, CCL11, CCL17, CCL19, CXCL9, CXCL10, CXCL11, IFNs, IL-1β, IL-6, TNF-α, IL-2, IL-4, IL-10, IL-17, TGF- α or CHI3L1 may be utilized, along with the existing biomarkers such as anti-dsDNA Ab in blood, total IgG in blood, and proteinuria levels, to predict the therapeutic effect of mesenchymal stem cells on systemic lupus erythematosus.

### Brief Description of Drawings

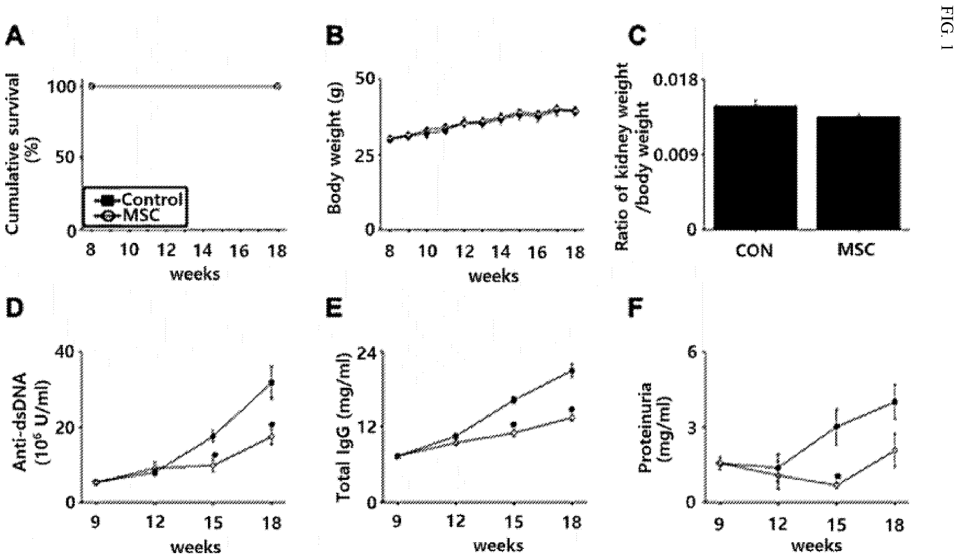

FIG. 1 illustrates a result obtained by confirming the therapeutic effect of MRL/lpr mice by the administration of mesenchymal stem cells. MRL/lpr mice were administered with mesenchymal stem cells at the age of 12 and 15 weeks. (a) is a result obtained by measuring the survival rate, (b) is a result obtained by measuring the body weight, (c) is a result obtained by measuring the weight of the kidney, (d) is a result obtained by measuring anti-dsDNA in serum, (e) is a result obtained by measuring total IgG, and (f) is a result obtained by measuring proteinuria in urine.

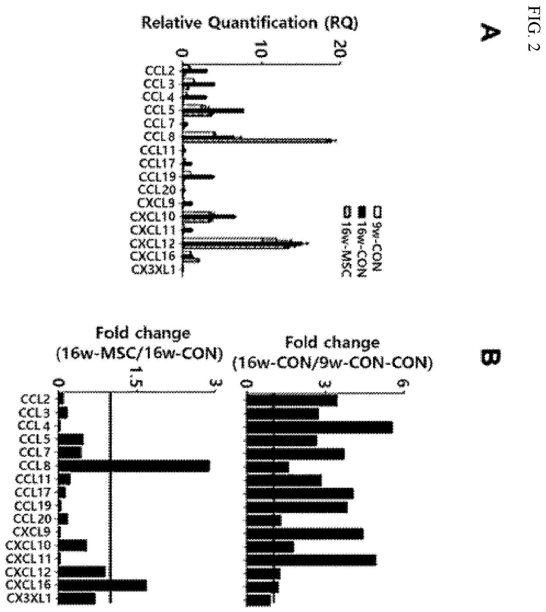

FIG. 2 illustrates a result obtained by confirming the chemokine changes in the kidney of MRL/lpr mice by the administration of mesenchymal stem cells. MRL/lpr mice were administered twice with mesenchymal stem cells, and an autopsy was performed at the age of 16 weeks. (a) is a result obtained by calculating as a relative quantitative value based on the expression level of CCL2 in the kidney tissue of the 9-week-old control group, and (b) is a result obtained by calculating the change in the value at the age of 16 weeks compared to the value at the age of 9 weeks or the change in the value of the group administered with mesenchymal stem cells compared to the control group at the age of 16 weeks.

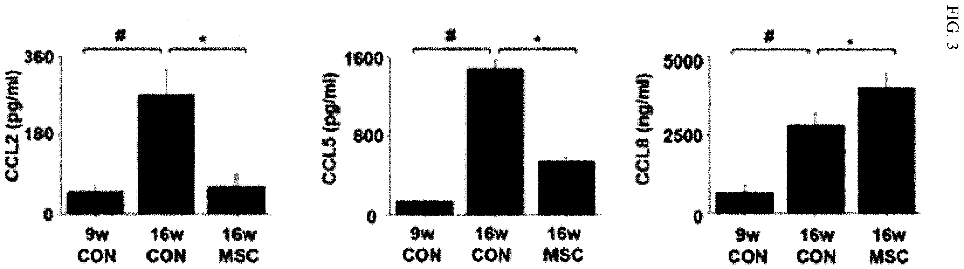

FIG. 3 illustrates a result obtained by confirming the chemokine changes in the serum of MRL/lpr mice by the administration of mesenchymal stem cells. MRL/lpr mice were administered twice with mesenchymal stem cells, and an autopsy was performed at the age of 16 weeks, and the serum was used to measure each chemokine.

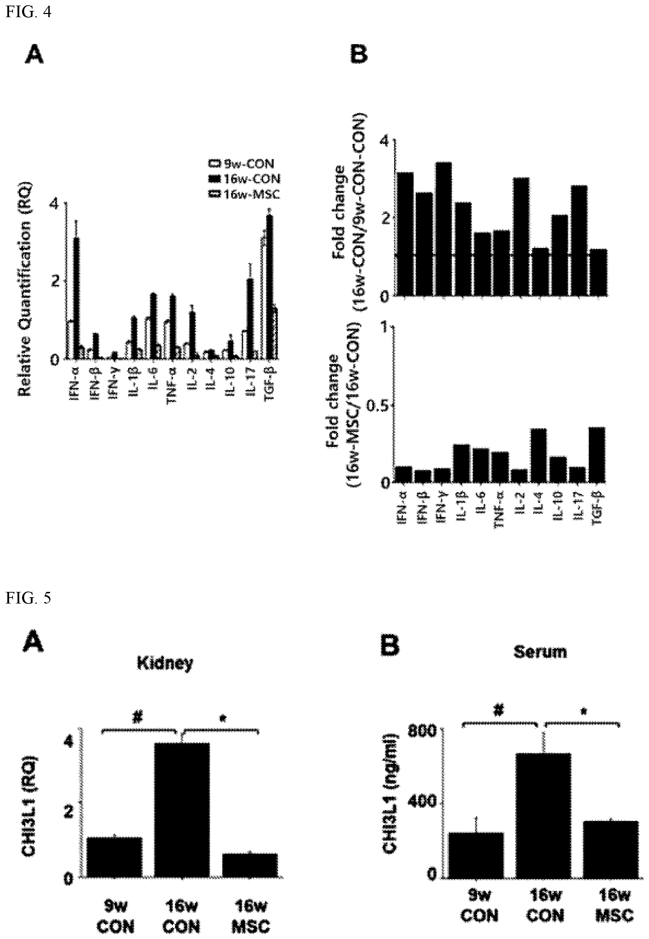

FIG. 4 illustrates a result obtained by confirming the cytokine changes in the kidney of MRL/lpr mice by the administration of mesenchymal stem cells. MRL/lpr mice were administered twice with mesenchymal stem cells, and an autopsy was performed at the age of 16 weeks. (a) is a result obtained by calculating as a relative quantitative value based on the expression level of IFN-α in the kidney tissue of the 9-week-old control group, and (b) is a result obtained by calculating the change in the value at the age of 16 weeks compared to the value at the age of 9 weeks or the change in the value of the group administered with mesenchymal stem cells compared to the control group at the age of 16 weeks.
FIG. 5 illustrates a result obtained by confirming the CHI3L1 changes in MRL/lpr mice by the administration of mesenchymal stem cells. MRL/lpr mice were administered twice with mesenchymal stem cells, and an autopsy was performed at the age of 16 weeks. (a) is a result obtained by calculating as a relative quantitative value based on the expression level of CHI3L1 in the kidney tissue of the 9-week-old control group, and (b) is a result obtained by measuring CHI3L1 using the serum.

### Mode for Carrying out the Invention

Hereinafter, the present invention will be described in more detail.

The present inventors measured the therapeutic effect of mesenchymal stem cells in a systemic lupus erythematosus disease animal model using the existing biomarkers (survival rate, anti-dsDNA Ab in blood, total IgG in blood, and proteinuria), and confirmed that new biomarkers (chemokine, cytokine, and CHI3L1) may be also measured. Based on the above, the present inventors completed the present invention.

The present invention may provide a biomarker composition for predicting the therapeutic effect of mesenchymal stem cells on systemic lupus erythematosus, comprising an agent for measuring an expression level of the mRNA or protein of CCL2, CCL3, CCL4, CCL5, CCL8, CCL7, CCL11, CCL17, CCL19, CXCL9, CXCL10, CXCL11, IFNs, IL-1β, IL-6, TNF-α, IL-2, IL-4, IL-10, IL-17, TGF-α or CHI3L1.

It was confirmed that when systemic lupus erythematosus progressed in MRL/lpr mice, anti-dsDNA Ab in blood, total IgG in blood, and proteinuria levels were increased, and ultimately the mice died. It was confirmed that when mesenchymal stem cells were administered to these mice, anti-dsDNAAb in blood, total IgG in blood, and proteinuria levels were reduced. Therefore, it was confirmed that these existing biomarkers might confirm the therapeutic effect of mesenchymal stem cells on systemic lupus erythematosus (FIG. 1).

Furthermore, the present inventors confirmed that when systemic lupus erythematosus progressed in MRL/lpr mice, the expression levels of various chemokines, cytokines, and CHI3L1 were changed (increased or reduced) and were regulated by the administration of mesenchymal stem cells (FIGs. 2 to 5). Typically, it was confirmed that mesenchymal stem cells reduced the expression of CCL2 and CCL5, increased the expression level of CCL8, reduced the expression of IL-1β, and reduced the expression of CHI3L1.

Through this, it was confirmed that in order to predict the therapeutic effect of mesenchymal stem cells on systemic lupus erythematosus, CCL2, CCL5, CCL8, IL-1β, and CHI3L1 may be utilized along with the existing biomarkers such as anti-dsDNA Ab in blood, total IgG in blood, and proteinuria levels.

As used herein, the term "agent for measuring an expression level of the mRNA or protein" refers to a molecule that may be used for detection and/or quantification of a marker by determining an expression level of the mRNA or protein of CCL2, CCL3, CCL4, CCL5, CCL8, CCL7, CCL11, CCL17, CCL19, CXCL9, CXCL10, CXCL11, IFNs, IL-1β, IL-6, TNF-α, IL-2, IL-4, IL-10, IL-17, TGF-α or CHI3L1, which is a marker that increases or decreases after treatment with mesenchymal stem cells in a patient with systemic lupus erythematosus. Specifically, the agent for measuring the mRNA expression level of the gene may be a primer pair, a probe or an antisense nucleotide that specifically binds to the mRNA. In this regard, since the nucleic acid information of the genes is known in genebank, etc., those of ordinary skill in the art may design a primer or a probe that specifically amplifies a specific region of these genes based on the sequence.

As used herein, the term "primer pair" includes all combinations of primer pairs consisting of forward and reverse primers recognizing a target gene sequence, and specifically, a primer pair that provides analysis results with specificity and sensitivity. Since the nucleic acid sequence of the primer is a sequence that is inconsistent with a non-target sequence present in the sample, high specificity may be conferred when the primer amplifies only the target gene sequence containing the complementary primer binding site and does not cause non-specific amplification.

As used herein, the term "probe" refers to a material capable of specifically binding to a target material to be detected in a sample, and refers to a material capable of specifically confirming the presence of a target material in the sample through the binding. The type of a probe molecule is not limited as a material commonly used in the art, but it may be preferably PNA (peptide nucleic acid), LNA (locked nucleic acid), peptide, polypeptide, protein, RNA or DNA. More specifically, the probe is a biomaterial derived from an organism or similar thereto, or includes one manufactured *ex vivo.* For example, the probe may be enzymes, proteins, antibodies, microorganisms, animal and plant cells and organs, nerve cells, DNA, and RNA, and DNA includes cDNA, genomic DNA, and oligonucleotides, RNA includes genomic RNA, mRNA, and oligonucleotides, and examples of proteins may include antibodies, antigens, enzymes, peptides, and the like.

As used herein, the term "antisense oligonucleotide" refers to DNA or RNA or a derivative thereof containing a nucleic acid sequence complementary to a specific mRNA sequence, which binds to a complementary sequence in mRNA and acts to inhibit the translation of mRNA into protein. An antisense oligonucleotide sequence refers to a DNA or RNA sequence that is complementary to mRNA of the genes and is capable of binding to the mRNA. This may inhibit the translation, translocation into the cytoplasm, maturation or essential activity for all other overall biological functions of the gene mRNA. The length of the antisense oligonucleotide may be 6 to 100 base pair (bp), preferably 8 to 60 bp, more preferably 10 to 40 bp. The antisense oligonucleotide may be synthesized *in vitro* by a conventional method and administered *in vivo,* or the antisense oligonucleotide may be synthesized *in vivo.* One example of synthesizing an antisense oligonucleotide *in vitro* is to use RNA polymerase I. One example of synthesizing an antisense RNA *in vivo* is to transcribe an antisense RNA by using a vector in which the origin of a multiple cloning site (MCS) is in the opposite direction. Preferably, the antisense RNA is not translated into a peptide sequence by allowing a translation stop codon to be present in the sequence.

The agent for measuring the protein expression level may be an antibody that specifically binds to the protein or a fragment of the protein, and the antibody may be a polyclonal antibody or a monoclonal antibody.

As used herein, the term "antibody" may refer to a specific protein molecule directed against an antigenic region. For the purpose of the present invention, an antibody refers to an antibody that specifically binds to a marker protein, and includes a polyclonal antibody, a monoclonal antibody and a recombinant antibody. An antibody may be readily prepared using techniques well known in the art. In addition, the antibody of the present specification includes a complete form having two full length light chains and two full length heavy chains, as well as functional fragments of the antibody molecule. A functional fragment of the antibody molecule refers to a fragment having at least an antigen binding function, and includes Fab, F (ab'), F (ab')₂ and Fv, etc.

The present invention provides a kit for predicting the therapeutic effect of mesenchymal stem cells on systemic lupus erythematosus, comprising the composition. Specific details of the composition are as described above. The kit may predict the therapeutic effect of mesenchymal stem cells on systemic lupus erythematosus by measuring the protein expression level of CCL2, CCL3, CCL4, CCL5, CCL8, CCL7, CCL11, CCL17, CCL19, CXCL9, CXCL10, CXCL11, IFNs, IL-1β, IL-6, TNF-α, IL-2, IL-4, IL-10, IL-17, TGF-α or CHI3L1, which is a marker for predicting the therapeutic effect of mesenchymal stem cells on systemic lupus erythematosus, or an expression level of the mRNA or protein of the gene. The kit may comprise an agent for measuring the mRNA expression level of a marker gene for predicting the therapeutic effect of mesenchymal stem cells in a patient with systemic lupus erythematosus, for example, a primer pair, a probe or an antisense nucleotide that specifically binds to the gene, and may comprise an agent for measuring the protein expression level, for example, an antibody that specifically binds to the marker protein or an antigen binding fragment of the antibody.

The kit may be a RT-PCR (reverse transcription polymerase chain reaction) kit, a DNA chip kit, an ELISA (enzyme-linked immunosorbent assay) kit, a protein chip kit, a rapid kit or an MRM (multiple reaction monitoring) kit.

The kit for predicting the therapeutic effect of mesenchymal stem cells on systemic lupus erythematosus may further include one or more other component compositions, solutions or devices suitable for the analysis method. For example, the diagnostic kit may be a diagnostic kit comprising essential elements necessary for performing a reverse transcription polymerase chain reaction. The reverse transcription polymerase chain reaction kit includes each primer pair specific for a marker gene. The primer is a nucleotide having a sequence specific to the nucleic acid sequence of each gene, and has a length of about 7 bp to 50 bp, more preferably a length of about 10 bp to 30 bp. In addition, it may include a primer specific for the nucleic acid sequence of a control gene. In addition, the reverse transcription polymerase chain reaction kit may include test tubes or other proper containers, reaction buffers (varying pH and magnesium concentrations), deoxynucleotides (dNTPs), enzymes such as Taq-polymerase and reverse transcriptase, DNAse, RNAse inhibitors, DEPC-water, sterile water, and the like. In addition, for example, the DNA chip kit may include a substrate to which cDNA or oligonucleotide corresponding to a gene or a fragment thereof is attached, and reagents, agents and enzymes for preparing a fluorescently labeled probe, and the like. In addition, substrate may include cDNA or oligonucleotide corresponding to a control gene or a fragment thereof. In addition, for example, the kit may be a diagnostic kit comprising essential elements necessary for performing ELISA. The ELISA kit includes an antibody specific for the protein. Antibodies may be antibodies having high specificity and affinity for each marker protein and little cross-reactivity with other proteins, and may be monoclonal antibodies, polyclonal antibodies or recombinant antibodies. In addition, the ELISA kit may include an antibody specific for a control protein. In addition, the ELISA kit may include reagents capable of detecting bound antibodies, for example, labeled secondary antibodies, chromophores, enzymes (for example, conjugated with an antibody) and substrates thereof or other materials capable of binding an antibody and the like. In addition, for example, the kit may be a rapid kit comprising essential elements necessary for performing a rapid assay that may determine the analysis result. The rapid kit includes an antibody specific for the protein. Antibodies may be antibodies having high specificity and affinity for each marker protein and little cross-reactivity with other proteins, and may be monoclonal antibodies, polyclonal antibodies or recombinant antibodies. In addition, the rapid kit may include an antibody specific for a control protein. In addition, the rapid kit may include reagents capable of detecting bound antibody, for example, a nitrocellulose membrane to which a specific antibody and a secondary antibody are immobilized, a membrane bound to an antibody bound bead, other materials such as an absorbent pad and a sample pad, and the like. In addition, for example, the kit may be an MRM (multiple reaction monitoring) kit in MS/MS mode comprising essential elements necessary for performing mass spectrometry. While SIM (selected ion monitoring) is a method that uses ions generated by one collision at the source part of the mass spectrometer, MRM is a method of selecting a specific ion from among the broken ions once more, passing it through another continuously connected MS source once more, colliding it to obtain the ions, and then using the ions among them. The MRM (multiple reaction monitoring) analysis methods may compare the protein expression level of the normal control group or the same subject with the protein expression level of a subject who has been treated with mesenchymal stem cells, and may also predict the therapeutic effect of mesenchymal stem cells on systemic lupus erythematosus by determining whether there is a significant increase or decrease in the expression level from the gene into the protein in a marker for predicting the therapeutic effect of mesenchymal stem cells on systemic lupus erythematosus.

The present invention may also provide a method of providing information for predicting the therapeutic effect of mesenchymal stem cells on systemic lupus erythematosus, comprising: measuring an expression level of the mRNA or protein of CCL2, CCL3, CCL4, CCL5, CCL8, CCL7, CCL11, CCL17, CCL19, CXCL9, CXCL10, CXCL11, IFNs, IL-1β, IL-6, TNF-α, IL-2, IL-4, IL-10, IL-17, TGF-α or CHI3L1 from a sample; and comparing the measured expression level to the expression level of a control group.

The sample may be obtained from a patient with systemic lupus erythematosus who has been treated with mesenchymal stem cells.

The method of providing the information comprises measuring an expression level of the mRNA or protein of CCL2, CCL3, CCL4, CCL5, CCL8, CCL7, CCL11, CCL17, CCL19, CXCL9, CXCL10, CXCL11, IFNs, IL-1β, IL-6, TNF-α, IL-2, IL-4, IL-10, IL-17, TGF-α or CHI3L1 in a biological sample isolated from a subject. Specifically, the expression level of mRNA or protein may be determined by measuring the mRNA expression level of the marker gene or the expression level of the protein encoded by the gene. Isolation of mRNA or protein from a sample of a subject may be appropriately performed by those of ordinary skill in the art according to methods known in the art. For example, after homogenizing the kidney tissue of a subject with a buffer for extracting protein or a buffer for extracting nucleic acid, and then centrifuging it, the obtained supernatant may be used as a sample for the subject. The subject may include a vertebrate, a mammal, or a human (Homo sapiens). The sample may be any one or more selected from the group consisting of tissue, cells, whole blood, serum and plasma. For example, it may use reverse transcription polymerase chain reaction (RT-PCR), competitive reverse transcription polymerase chain reaction (competitive RT-PCR), real time reverse transcription polymerase chain reaction (real time quantitative RT-PCR), quantitative polymerase chain reaction (quantitative RT-PCR), RNase protection method, Northern blotting or DNA chip technology. In addition, the measurement of the protein expression level may use, for example, Western blotting, immunohistochemical staining, immunoprecipitation assay, complement fixation assay or immunofluorescence.

The method may determine that the mesenchymal stem cells have the therapeutic effect or the excellent therapeutic effect when the gene expression level of CCL2, CCL3, CCL4, CCL5, CCL7, CCL11, CCL17, CCL19, CXCL9, CXCL10, CXCL11, IFNs, IL-1β, IL-6, TNF-α, IL-2, IL-4, IL-10, IL-17, TGF-α or CHI3L1 is measured to be low compared to that of the control sample. The control sample is a patient with systemic lupus erythematosus who has not been treated with mesenchymal stem cells.

The method may determine that the mesenchymal stem cells have the therapeutic effect or the excellent therapeutic effect when the gene expression level of CCL8 is measured to be high compared to that of the control sample. The control sample is a patient with systemic lupus erythematosus who has not been treated with mesenchymal stem cells.

Hereinafter, the present invention will be described in more detail through examples. The following examples are only preferred specific examples of the present invention, and the scope of the present invention is not limited to the scope of the following examples.

### <Example 1> Design of animal experiment

MRL/lpr mouse is a systemic lupus erythematosus disease animal model in which systemic lupus erythematosus develops at the age of 12 weeks and at least 50% of the mice die at the age of 20 weeks. The age of 9 weeks was regarded as pre-onset, and the age of 16 weeks or older was determined as mid/late onset, and applied to the test model. Female MRL/MpJ-Faslpr/J (hereinafter referred to as MRL/lpr) mice were purchased from The Jackson Laboratory. The breeding environment of the mice was maintained at a specific pathogen-free (SPF) state with a temperature of 21 ∼ 24 °C, a humidity of 40 ~ 60%, and a light/dark cycle of 12 hours (light on: 08 o'clock, light off: 20 o'clock). Solid feed and drinking water were sterilized and fed freely, and all experimental animals were acclimatized in the animal room for 1 week before use. Human mesenchymal stem cells were purchased from CORESTEM, Inc. Normal bone marrow derived mesenchymal stem cells were cultured using CSBM-A06 medium, and passage 4 (p4) cells were used for the experiment. MRL/lpr mice were administered twice with human mesenchymal stem cells at a concentration of 4×10⁶ cells/mouse into the tail vein at the age of 12 and 15 weeks of the mice. Therefore, the survival rate and body weight were measured weekly. There was no difference in changes in the survival rate and weight (FIGs. 1A and 1B). The kidney weight compared to the weight after an autopsy was decreased in the group administered with mesenchymal stem cells compared to the control group (FIG. 1C). While performing an autopsy at the age of 16 or 18 weeks, urine, serum, and kidney were separated and used for analysis assay.

### <Example 2> Measurement of Anti-dsDNA

Anti-dsDNA was measured using the serum isolated from the animal model of Example 1, and it was performed according to the test method provided by Anti-dsDNA Ab ELISA (Alpha diagnostic international). The mouse serum was diluted with Working Sample Diluent (WSD), and then 100 µl of the diluted sample was treated in an anti-dsDNA coated well and reacted at room temperature for 60 minutes. Thereafter, it was washed 4 times with 200 µl of washing solution, and 100 µl of Anti-Mouse IgG HRP was added to each well and reacted at room temperature. After 30 minutes, it was washed 4 times with 200 µl of washing solution. 100 µl of TMB solution was added to each well, then the color was observed, and then, 10 minutes later, 100 µl of stop solution was added to stop the reaction. Finally, the OD value was measured at 450 nm. The concentration of anti-dsDNAAb in blood of MRL/lpr mice was increased after the age of 12 weeks and decreased when mesenchymal stem cells were administered (FIG. ID).

### <Example 3> Measurement of total IgG

Total IgG was measured using the serum isolated from the animal model of Example 1, and it was performed according to the test method provided by Total IgG ELISA (Thermo Fisher Scientific). In 96 wells for ELISA, capture antibody and coating buffer were diluted at a ratio of 1:250 and coated for 4 to 18 hours. It was washed twice with 200 µl of washing solution, and 250 µl of blocking buffer was added to the well and then reacted at room temperature for 2 hours. Thereafter, it was washed twice with 200 µl of washing solution, and the mouse serum was diluted in assay buffer A (1×) to prepare a sample. 90 µl of assay Buffer A (1×), 50 µl of detection-antibody and 10 µl of standard or sample were added to each well and reacted at room temperature for 3 hours. After the reaction was completed, it was washed with a washing solution. 100 µl of substrate solution was added to each well, and then reacted for 5 minutes, and 100 µl of stop solution was added to stop the reaction. Finally, the value was measured at 450 nm to 570 nm. The concentration of total IgG in blood of MRL/lpr mice was also increased from the age of 12 weeks and decreased when mesenchymal stem cells were administered (FIG. 1E).

### <Example 4> Measurement of proteinuria

Proteinuria was measured using the isolated urine, and it was performed according to the test method provided by Pierce 660 nm Protein Assay (Thermo Fisher Scientific). The mouse urine was diluted with PBS, and then 10 µl of the diluted sample was reacted with 150 µl of protein assay reagent at room temperature, and 10 minutes later, the OD value was measured at 660 nm. Proteinuria using urine was also decreased in the group administered with mesenchymal stem cells compared to the control group (FIG. 1F). In MRL/lpr mice, it was confirmed that mesenchymal stem cells had the effect of alleviating the onset of systemic lupus erythematosus.

### <Example 5> Quantitative real-time PCR (qPCR)

In order to confirm the chemokine network, the gene expression of chemokines was measured using the kidney of MRL/lpr mice. Total RNA was isolated from the kidney tissue using TRIZOL Reagent (Invitrogen). cDNA was synthesized at a concentration of 1 µg of total RNA. qPCR was performed on the synthesized cDNA using a SYBR green probe at a concentration of 50 ng. The measured chemokines are CCL2, CCL3, CCL4, CCL5, CCL7, CCL8, CCL11, CCL17, CCL19, CCL20, CXCL9, CXCL10, CXCL11, CXCL12, CXCL16, and CX3CL1, most of which are known to be chemokines associated with inflammation. As a result of the test, MRL/lpr mice showed the increased expression of chemokine at the age of 16 weeks compared to the age of 9 weeks, which was determined to be phenomenon caused by the onset of systemic lupus erythematosus. It was confirmed that the group administered with mesenchymal stem cells showed the decreased expression of chemokine (FIG. 2A). It was confirmed that CCL2, 3, 4, 5 and CXCL9, 10 associated with inflammation were reduced when mesenchymal stem cells were administered, but specifically, CCL8 was increased according to the age of mice and was also increased further when mesenchymal stem cells were administered. All chemokines except for CX3CL1 were increased at the age of 16 weeks compared to the age of 9 weeks even in the change in values at the age of 9 weeks and 16 weeks. As a result of analyzing the change in values of the control group and the group administered with mesenchymal stem cells at the age of 16 weeks, most of the chemokines were decreased, but only the chemokines of CCL8 and CXCL16 were increased when mesenchymal stem cells were administered (FIG. 2B).

### <Example 6> Measurement of chemokine

In order to confirm the systemic immune response based on the analysis of gene expression in the kidney, changes in chemokines in serum were determined. The inflammatory chemokines CCL2 and CCL5 and CCL8, which responded specifically to the administration of mesenchymal stem cells, were measured. It was performed according to the test method provided by measurement kit (R&D SYSTEMS) of CCL2, CCL5 and CCL8. Capture antibody was diluted in PBS according to the concentration in the kit in 96 wells for ELISA and coated at room temperature for 18 hours. It was washed twice with 200 µl of washing solution, and 300 µl of reagent diluent was added to the well and then reacted at room temperature 1 hour. Thereafter, it was washed twice with 200 µl of washing solution, and the mouse serum was diluted in reagent diluent (1×) to prepare a sample. 100 µl of standard or sample was added to each well and reacted at room temperature for 2 hours. After the reaction was completed, it was washed with a washing solution. Detection antibody was diluted in reagent diluent according to the concentration in the kit and reacted for 2 hours. After the reaction was completed, it was washed with a washing solution. Streptavidin-HRP was diluted 40-fold in reagent diluent and reacted for 20 minutes. After the reaction was completed, it was washed with a washing solution. 100 µl of substrate solution was added to each well, and then reacted for 20 minutes, and 50 µl of stop solution was added to stop the reaction. Finally, the value was measured at 450 nm - 540 nm.

As in gene expression, CCL2, CCL5, and CCL8 in serum were increased with the onset of systemic lupus erythematosus (FIG. 3). It was confirmed that CCL2 and CCL5 were decreased but CCL8 was further increased even in serum when mesenchymal stem cells were administered. It was confirmed that it could be utilized as a biomarker for predicting the onset of systemic lupus erythematosus and the effect of mesenchymal stem cells when CCL2, CCL5, and CCL8 were measured in systemic lupus erythematosus.

### <Example 7> Measurement of expression level of IFN

The gene expression of cytokines was measured using the kidney of MRL/lpr mice at the age of 9 weeks before the onset and at the age of 16 weeks during the mid-onset stage. Total RNA was isolated from the kidney tissue using TRIZOL Reagent (Invitrogen). cDNA was synthesized at a concentration of 1 µg of total RNA. qPCR was performed on the synthesized cDNA using a SYBR green probe at a concentration of 50 ng. In MRL/lpr mice, it was confirmed that all cytokines were increased as the age of mice was increased, and decreased when mesenchymal stem cells were administered (FIG. 4A). Even in the analysis of the change in values at the age of 9 weeks and 16 weeks, the gene expression rate of the IFN family was the highest compared to that of other cytokines, and was increased three times or more at the age of 16 weeks compared to the age of 9 weeks (FIG. 4B). Therefore, it was confirmed once again that IFN plays a major role in the pathogenesis of systemic lupus erythematosus.

### <Example 8> ELISA Measurement of CHI3L1

By confirming whether CHI3L1, which is associated with inflammation and induction of cell proliferation, also responds to systemic lupus erythematosus, we examined whether it can be applied as a biomarker for predicting the onset of systemic lupus erythematosus and the therapeutic effect by mesenchymal stem cells. CHI3L1 was measured using the isolated serum and it was performed according to the test method provided by Mouse Chitinase 3-like 1 (R&D SYSTEM). Serum was diluted 500-fold with assay diluent before use. In MRL/lpr mice, the gene expression of CHI3L1 in the kidney (FIG. 5A) and the production of CHI3L1 in the serum (FIG. 5B) were increased at the age of 16 weeks compared to the age of 9 weeks. Therefore, it was confirmed that when mesenchymal stem cells were administered, the gene expression of CHI3L1 in the kidney and the production of CHI3L1 in the serum were statistically significantly decreased. Therefore, CHI3L1 could be utilized as a potential biomarker for predicting the onset of systemic lupus erythematosus and the therapeutic effect by mesenchymal stem cells.

## Claims

1. A biomarker composition for predicting the therapeutic effect of mesenchymal stem cells on systemic lupus erythematosus, comprising an agent for measuring an expression level of the mRNA or protein of CCL2, CCL3, CCL4, CCL5, CCL8, CCL7, CCL11, CCL17, CCL19, CXCL9, CXCL10, CXCL11, IFNs, IL-1β, IL-6, TNF-α, IL-2, IL-4, IL-10, IL-17, TGF-α or CHI3L1 from a sample.

2. The biomarker composition for predicting the therapeutic effect of mesenchymal stem cells on systemic lupus erythematosus according to claim 1, wherein the agent for measuring the mRNA expression level is a primer pair, a probe or an antisense nucleotide that specifically binds to the mRNA.

3. The biomarker composition for predicting the therapeutic effect of mesenchymal stem cells on systemic lupus erythematosus according to claim 1, wherein the agent for measuring the protein expression level is an antibody that specifically binds to the protein or a fragment of the protein.

4. A kit for predicting the therapeutic effect of mesenchymal stem cells on systemic lupus erythematosus, comprising the composition of claim 1.

5. The kit for predicting the therapeutic effect of mesenchymal stem cells on systemic lupus erythematosus according to claim 4, wherein the kit is a RT-PCR (reverse transcription polymerase chain reaction) kit, a DNA chip kit, an ELISA (enzyme-linked immunosorbent assay) kit, a protein chip kit, a rapid kit or an MRM (multiple reaction monitoring) kit.

6. A method of providing information for predicting the therapeutic effect of mesenchymal stem cells on systemic lupus erythematosus, comprising:
measuring an expression level of the mRNA or protein of CCL2, CCL3, CCL4, CCL5, CCL8, CCL7, CCL11, CCL17, CCL19, CXCL9, CXCL10, CXCL11, IFNs, IL-1β, IL-6, TNF-α, IL-2, IL-4, IL-10, IL-17, TGF-α or CHI3L1 from a sample; and
comparing the measured expression level to the expression level of a control group.

7. The method of providing information for predicting the therapeutic effect of mesenchymal stem cells on systemic lupus erythematosus according to claim 6, wherein the measurement of the mRNA expression level uses reverse transcription polymerase chain reaction (RT-PCR), competitive reverse transcription polymerase chain reaction (competitive RT-PCR), real time reverse transcription polymerase chain reaction (real time quantitative RT-PCR), quantitative polymerase chain reaction (quantitative RT-PCR), RNase protection method, Northern blotting or DNA chip technology.

8. The method of providing information for predicting the therapeutic effect of mesenchymal stem cells on systemic lupus erythematosus according to claim 6, wherein the measurement of the protein expression level uses Western blotting, immunohistochemical staining, immunoprecipitation assay, complement fixation assay or immunofluorescence.

9. The method of providing information for predicting the therapeutic effect of mesenchymal stem cells on systemic lupus erythematosus according to claim 6, wherein the method determines that the mesenchymal stem cells have the therapeutic effect or the excellent therapeutic effect when the gene expression level of CCL2, CCL3, CCL4, CCL5, CCL7, CCL11, CCL17, CCL19, CXCL9, CXCL10, CXCL11, IFNs, IL-1β, IL-6, TNF-α, IL-2, IL-4, IL-10, IL-17, TGF-α or CHI3L1 from the sample is measured to be low compared to that of the control sample.

10. The method of providing information for predicting the therapeutic effect of mesenchymal stem cells on systemic lupus erythematosus according to claim 6, wherein the method determines that the mesenchymal stem cells have the therapeutic effect or the excellent therapeutic effect when the gene expression level of CCL8 is measured to be high compared to that of the control sample.
